# EUROPEAN PATENT SPECIFICATION

(11) **EP 3 834 813 B1**
(45) Date of publication and mention of the grant of the patent: **20.08.2025**
(21) Application number: 20212002.8
(22) Date of filing: 04.12.2020
(51) Int. Cl.: A61K 8/9789, A61K 8/73, A61Q 19/08

(54) **COSMETIC COMPOSITIONS FOR PREVENTING AND TREATING CUTANEOUS AGEING**
KOSMETISCHE ZUSAMMENSETZUNGEN ZUR VERHINDERUNG UND BEHANDLUNG VON KUTANEM ALTERUNG
COMPOSITIONS COSMÉTIQUES POUR LA PRÉVENTION ET LE TRAITEMENT DU VIEILLISSEMENT CUTANÉ

(30) Priority: 09.12.2019 IT 201900023349
(43) Date of publication of application: 16.06.2021
(73) Proprietor: Unifarco S.p.A., 32035 Santa Giustina (BL) (IT)
(72) Inventor: Baratto, Giovanni, 32035 Santa Giustina BL (IT)
(74) Representative: Perani & Partners S.p.A.

(56) References cited:
- EP-A1- 2 037 874
- WO-A2-2008/003321
- WO-A2-2014/044808
- CN-A- 107 362 102
- KR-A- 20150 081 892
- US-A- 4 855 137
- DATABASE GNPD [online] MINTEL; 28 April 2015 (2015-04-28), ANONYMOUS: "White Serum", XP055694780, retrieved from www.gnpd.com Database accession no. 3147359
- DATABASE GNPD [online] MINTEL; 29 April 2019 (2019-04-29), ANONYMOUS: "Whitening Jelly Essence", XP055694773, retrieved from www.gnpd.com Database accession no. 6512023
- DATABASE GNPD [online] MINTEL; 14 December 2017 (2017-12-14), ANONYMOUS: "Serum", XP055694775, retrieved from www.gnpd.com Database accession no. 5316383
- DATABASE GNPD [online] MINTEL; 12 June 2017 (2017-06-12), ANONYMOUS: "Moist Lotion", XP055694768, retrieved from www.gnpd.com Database accession no. 4882669

## Description

### FIELD OF THE INVENTION

The present invention relates to cosmetic compositions comprising hyaluronic acid and an osmoprotectant for the prevention and treatment of extrinsic and intrinsic skin aging.

### BACKGROUND ART

Skin is the organ which completely covers the surface of the body with an extension of about 150-200 cm² in the adult subject, and represents the border between our body and the outside world. Several important functions are attributed thereto: defence against chemical, physical and biological agents; perception of the outside world, thanks to the presence of a rich nervous network; synthesis of numerous substances (vitamin D, antimicrobials, moisturisers). Skin also contributes to thermoregulation and immune response and is a route of elimination, through sweating, not only of natural waste substances, but also of drugs and toxins. Finally, but of fundamental importance for survival, skin has the ability to retain water in our body, which has a much higher percentage than the external environment. Thanks to the particular morphological structure thereof, skin retains water inside, while communicating and adapting to external changes and thus allows us to live in an environment with extremely variable conditions.

Skin aging is a physiological process immediately observable by the person and is influenced by both intrinsic factors (genetic heritage, endocrine system) and extrinsic factors (exposure to the sun without protection, pollution, incorrect lifestyle).

Intrinsic skin aging consists of a progressive loss of cell matrix organization at the level of both the dermis and epidermis. The epidermis consists of 80% keratinocytes, which are cells responsible for the continuous renewal thereof. As age progresses, several more or less profound changes occur: cell renewal slows down and, consequently, the epidermis becomes thinner. In addition, the lipid barrier between cells becomes insufficient and no longer functions properly. At a deeper level, in the dermis, fibroblasts decrease in number and begin to synthesize less collagen and elastin fibres, causing a change in skin tone and elasticity. The production of glycosaminoglycans (GAG) responsible for deep hydration is also reduced. Extrinsic skin aging is caused by many factors such as environmental pollution, alcohol consumption, drugs, and cigarette smoking which, above all of them, greatly reduces collagen synthesis. Among the environmental factors which cause skin aging, one of the most harmful is the incorrect exposure to solar radiation which determines an early aging process, especially by stimulating the production of free radicals, the main enemies of the skin.

In any case, both intrinsic and extrinsic aging cause structural changes in skin, and wrinkles are the most obvious sign of this.

Hyaluronic acid (HA) or better still the biologically acceptable salt thereof, sodium hyaluronate, is a polymer belonging to the heteropolysaccharides family called Glycosaminoglycans (GAG) and is formed by units of D-glucuronic acid and N-acetylglucosamine. HA is therefore an anionic unsulphated glycosaminoglycan, which is found in the epithelial, connective and nerve tissues of vertebrates. The synthesis thereof occurs on the inner side of the plasma membrane (rather than in the Golgi) and is not covalently bound to any protein. In addition, it can have different molecular weights, even very high compared to the other GAGS.

In fact, it is one of the main components of the extracellular matrix and, precisely for this reason, promotes both cell proliferation and migration.

Hyaluronic acid owes the effectiveness thereof to the extraordinary ability thereof to absorb and retain large amounts of water (up to about 1000 times the weight thereof) and acts as a cellular cementing agent, ensuring an excellent degree of hydration of the skin tissues: the higher the content thereof in the epidermis, the plumper, smoother and intensely hydrated the skin.

However, our body produces less with advancing age already from the age of 20, while after the age of 40 it reduces considerably with the result that the first signs of skin aging appear, such as loss of elasticity, firmness and even volume. As a person ages, the appearance of the face changes, it loses the natural fullness and elasticity thereof, the cheeks and cheekbones become empty. Applying this precious ingredient through cosmetic formulations that contain it as an active ingredient can help prevent and correct wrinkle imperfection.

However, the activity of exogenously administered hyaluronic acid can be counteracted by the endogenous activity of the enzyme hyaluronidase and other enzymes which reduce the ability to act thereof, in addition there is a need to modulate the hydration of hyaluronic acid and therefore the activity thereof over time.

Mintel GNPD RECORD ID3147359 "White serum" 28..04.2015, discloses a cosmetic composition comprising among the plethora of cosmetic ingredients the sodium sale of Hyaluronic acid the extract Helianthus tuberosus glycerine and propanediol.

Mintel GNPD RECORD ID65120223 Whitening Jelly 29.04. 2019 discloses a cosmetic composition comprising among the plethora of cosmetic ingredients the sodium salt of hyaluronic and the hydrolyzed form thereof, the extract Helianthus tuberosus, glycerine and propanediol.

Mintel GNPD RECORD ID 5316383 "Serum" 14.12.2017 discloses a cosmetic composition comprising HYALURONIC ACIDM EXTRACT OF Helianthus tuberosus glycerin and propanediol.

Mintel GNPD RECORD ID4882669 "Moist lotion"12.06.2017 discloses a cosmetic composition containing 3 types of hyaluronic acid and also the hyaluronic acid salt, the extract of Helianthus Tuberosus.

EP2037874 discloses a moisturizing cosmetic composition comprising two molecular fractions of HA having respectively a molecular weight ranging from 8000 to 100000 D and from 100,000 to 500,000 D.

WO2008003321 discloses a moisturizing cosmetic composition comprising two molecular fractions of HA having respectively a molecular weight ranging from 8000 to 100,000 D and from 500,000 to 1,100,000 D.

WO201404408 discloses a cosmetic composition comprising a mixture of 6 fraction of HA.

CN107362102 discloses a cosmetic composition comprising from 0.01 to 1 % of Jerusalem artichoke leaf extract.

KR20150081892 discloses the use of Helianthus tuberosus for improving skin moisturization and elasticity.

US4855137 discloses the use oof aerial shoot and/or tube of Helianthus tuberosus for skin calming and regeneration thereof.

### DISCLOSURE OF THE INVENTION

The applicant has now found that it is possible to obtain cosmetic compositions in which the activity of hyaluronic acid is enhanced by the fact that hyaluronic acid or a biologically acceptable salt thereof is associated with an osmoprotectant comprising an extract of *Helianthus tuberosus.*

Hyaluronic acid in contact with body fluids which essentially consist of water tends to hydrate and increase volume. The presence of this osmoprotectant helps stabilize the polymer in a more compact globular form, which is however capable of binding a normal amount of water. The globular structure of hyaluronic acid, more compact in the presence of the osmoprotectant, makes it less exposed to degradative phenomena which normally occur with a direct exposure of hyaluronic acid to an aqueous environment.

The object of the present invention are therefore cosmetic compositions as claimed in claim 1 comprising as active ingredient:.
a) hyaluronic acid or a biologically acceptable salt thereof,
b) an osmoprotectant comprising an extract of *Helianthus tuberosus,*
in combination with suitable excipients .

The extract of *Helianthus Tuberosus* obtained is a cosmetic-grade raw material which, in addition to being rich in polysaccharides, at low and medium molecular weight, also contains polyphenols.

The present invention thus relates to the use of said cosmetic compositions for the treatment and prevention of extrinsic and intrinsic skin aging.

For the purposes of the present invention, the definitions "comprising" and "containing" do not exclude the presence of additional components other than those mentioned after such definition.

The definition "consisting of" excludes the presence of additional components listed after such a definition.

In the cosmetic compositions object of the invention, the biologically acceptable salt is an alkali metal salt, and more preferably is sodium salt.
B) hyaluronic acid with a weight average molecular weight between 10000 and 1,000,000 Da,
C) hyaluronic acid with a weight average molecular weight > 1,000,000 Da,
or mixtures thereof, of at least two of the above types.

According to a preferred solution the cosmetic compositions object of the present invention comprise one or all three types of hyaluronic acid in concentrations between 0.01% and 1% by weight on the total weight of said cosmetic compositions.

Thereby, an action on the entire skin barrier is guaranteed.

In fact, by employing:
- a high molecular weight HA of type C), a lifting and moisturising action is obtained on the dermal surface,
- a medium molecular weight HA of type B), a moisturising and anti-wrinkle action is obtained at the dermis-epidermal junction;
- a low molecular weight HA of type A), a deeper moisturising action and stimulation of skin cell metabolism is obtained.

*Helianthus tuberosus,* commonly known as Jerusalem artichoke, belongs to the genus *Helianthus* of the botanical family *Asteraceae.*

It is a tuber plant which, unlike the potato tuber, does not contain starch but inulin at a rate of 14-16% in weight on the total weight of the plant. Because of the inulin content, Jerusalem artichoke tubers are often used in the food industry and distillery.

For the purposes of the present invention an extract of *Helianthus tuberosus,* also known as Topinambur, is meant, which is obtained only from the roots or tubers of said plant by a digestive process of the roots (tubers) processed so as to obtain a raw material of vegetable origin which is "cosmetic grade" and free of preservatives.

Preferably the extraction comprises the following steps: the fresh root is added with water respectively in a weight ratio between 1/1 and 1/6, preferably 1/5. These components are then mixed for 60 minutes at a temperature between 80°C and 100°C, allowed to digest for 2 hours at a temperature between 80°C and 100°C, possibly adding the water which has evaporated, thus obtaining the aqueous extract.

This type of extract is characterized by a colouration which could affect the organoleptic characteristics of the finished cosmetic product. Therefore, dilution is necessary before inserting the extract as it is in the cosmetic formula.

This type of extract is characterized by a colouration which could affect the organoleptic characteristics of the finished cosmetic product. Therefore, dilution is necessary before inserting the extract as it is in the cosmetic formula.

The diluent substance must be chosen so that it is compatible with use in cosmetic formulations, as required by Regulation 1223/2009, in addition to supporting the desired organoleptic characteristics and pH conditions in a cosmetic product.

Therefore, a post-extraction dilution step is included using different extract/diluent ratios, such a diluent also being chosen to respect the desired characteristics described above

Preferably the extract obtained is generally diluted with a polyol or a mixture of polyols, so that the diluent also performs the technical function of sequestrant of the aqueous phase, so as to prevent any microbial contamination.

Preferably the extract obtained is diluted with glycerine, propanediol or mixtures thereof.

Even more preferably the Heliantus tuberosus extract achieves optimal stability conditions with the mixture of glycerine and propanediol diluents, and, according to a particularly preferred solution with a glycerine/propanediol/extract weight ratio of *35*/*35*/*30. The Helianthus Tuberosus* extract thus obtained was analysed and, in addition to being rich in low and medium molecular weight polysaccharides, it also contains polyphenols such as chlorogenic acid, which allows an anti-aging effect to be obtained by working on the production of oxygen radicals (ROS) known to promote photo-aging induced by the exposure to UV rays. The cosmetic compositions can then be used for the treatment and prevention of extrinsic and intrinsic skin aging. The cosmetic compositions object of the present invention preferably comprise hyaluronic acid or component a) and osmoprotectant or component b) in a ratio comprised between 2:1 and 1:2, more preferably 1:1.

The cosmetic compositions according to the present invention comprise component a) in concentrations between 0.01 and 1% by weight on the total weight of said cosmetic compositions, component b) in concentrations between 0.01 and 1% by weight on the total weight of said compositions.

Some cosmetic compositions object of the present invention are given for illustrative but non-limiting purposes.

### FACIAL LOTION:

| **Component** | **range [% w/w]** |
|---|---|
| Aqua | as needed up to 100 |
| Humectants | 1-20 |
| Rheological modifiers | 1-5 |
| Emulsifiers | 0.5-3 |
| Preservatives | 0.1-1.5 |
| Perfume | 0.1-1 |
| **Hyaluronic acid** | **0.01-1** |
| **Helianthus Tuberosus extract** | **0.01-1** |

### O/A EMULSION:

| **Component** | **range [% w/w]** |
|---|---|
| Aqua | as needed up to 100 |
| Oils (vegetable or mineral) Waxes, fats (e.g., long chain alcohols) | 1-5 |
| Silicones | 1-10 |
| Humectants | 1-20 |
| Rheological modifiers | 1-5 |
| Emulsifiers | 0.5-3 |
| Preservatives | 0.1-1.5 |
| Perfume | 0.1-1 |
| **Hyaluronic acid** | **0.05-1** |
| **Helianthus Tuberosus extract** | **0.05-1** |

### SOLAR EMULSION:

| **Component** | **range [% w/w]** |
|---|---|
| Aqua | as needed up to 100 |
| Oils (vegetable or mineral) Waxes, fats (e.g., long chain alcohols) | 5-10 |
| UV filters | 20-25 |
| Silicones | 1-10 |
| Humectants | 1-20 |
| Rheological modifiers | 1-5 |
| Emulsifiers | 0.5-3 |
| Preservatives | 0.1-1.5 |
| Perfume | 0.1-1 |
| Chelating Agent | 0.1-0.3 |
| Antioxidants | 0.03-0.5 |
| **Hyaluronic acid** | **0.01-1** |
| **Helianthus Tuberosus extract** | **0.01-1** |

## Claims

1. Cosmetic compositions comprising as active principle:
a) hyaluronic acid or a biologically acceptable salt thereof,
b) an osmoprotector comprising an extract of Helianthus tuberosus in combination with suitable excipients, wherein
- component a) is in concentrations comprised between 0.01 and 1% by weight on the total weight of said cosmetic compositions,
- component b) is in concentrations comprised between 0.01 and 1% by weight on the total weight of said compositions;
- Helianthus tuberosus extract is obtained from roots or tubers of said plant by a digestive process.

2. Cosmetic compositions according to claim 1, wherein the biologically acceptable salt is a salt of an alkaline metal.

3. Cosmetic compositions according to claims 1 and 2, wherein the biologically acceptable salt is sodium salt.

4. Cosmetic compositions according to any one of claims from 1 to 3, wherein hyaluronic acid is selected from at least one of the following types:
A) hyaluronic acid with weight average molecular weight < 10,000 Da,
B) hyaluronic acid with a weight average molecular weight between 10000 and 1,000,000 Da,
C) hyaluronic acid with weight average molecular weight > 1,000,000 Da.

5. Cosmetic compositions according to any one of claims from 1 to 4, which include one or all three types of hyaluronic acid, A), B) and C), in concentrations such that the sum of the concentrations of said types of hyaluronic acid is comprised between 0.01% and 1% by weight on the total weight of said cosmetic compositions.

6. Cosmetic compositions according to any one of claims 1-5, wherein the weight ratio between components a) and b) is comprised between 1:2 and 2:1.

7. Cosmetic compositions according to any one of claims 1-6, wherein the weight ratio between components a) and b) is 1:1.

8. Cosmetic compositions according to any one of claims from 1 to 7, wherein component b) comprises the extract of Helianthus tuberosus and at least one diluent selected in the class of polyols.

9. Cosmetic compositions according to any one of claims from 1 to 8, wherein said polyols are selected from glycerine and propanediol and relative mixtures.

10. Cosmetic compositions according to any one of claims from 1 to 9, wherein said polyols are a mixture of glycerine and propanediol in the following weight ratios with respect to the extract glycerine /propanediol/extract respectively of 35/35/30.

11. Use of cosmetic compositions according to any one of claims 1-10 for the treatment and prevention of the extrinsic and intrinsic aging of the skin.

## Patentansprüche

1. Kosmetische Zusammensetzungen, umfassend als Wirkstoff:
a) Hyaluronsäure oder ein biologisch annehmbares Salz davon,
b) einem Osmoprotektor, umfassend einen Extrakt von Helianthus tuberosus in Kombination mit geeigneten Hilfsstoffen, wobei
- die Komponente a) in Konzentrationen zwischen 0,01 und 1 Gewichts- % bezogen auf das Gesamtgewicht der kosmetischen Zusammensetzungen ist,
- die Komponente b) in Konzentrationen zwischen 0,01 und 1 Gewichts- % bezogen auf das Gesamtgewicht der Zusammensetzungen ist;
- Helianthus tuberosus-Extrakt aus Wurzeln oder Knollen der Pflanze durch einen digestiven Prozess erlangt wird.

2. Kosmetische Zusammensetzungen nach Anspruch 1, wobei das biologisch annehmbare Salz ein Salz eines Alkalimetalls ist.

3. Kosmetische Zusammensetzungen nach den Ansprüchen 1 und 2, wobei das biologisch annehmbare Salz Natriumsalz ist.

4. Kosmetische Zusammensetzungen nach einem der Ansprüche 1 bis 3, wobei Hyaluronsäure ausgewählt ist aus mindestens einem der folgenden Typen:
A) Hyaluronsäure mit einem gewichtsmittleren Molekulargewicht <10.000 Da,
B) Hyaluronsäure mit einem gewichtsmittleren Molekulargewicht zwischen 10.000 und 1.000.000 Da,
C) Hyaluronsäure mit einem gewichtsmittleren Molekulargewicht >1.000.000 Da.

5. Kosmetische Zusammensetzungen nach einem der Ansprüche 1 bis 4, die eine oder alle drei Typen von Hyaluronsäure, A), B) und C), in Konzentrationen beinhalten, sodass die Summe der Konzentrationen der Typen von Hyaluronsäure zwischen 0,01 und 1 Gewichts-% bezogen auf das Gesamtgewicht der kosmetischen Zusammensetzungen liegt.

6. Kosmetische Zusammensetzungen nach einem der Ansprüche 1-5, wobei das Gewichtsverhältnis zwischen Komponente a) und b) zwischen 1:2 und 2:1 liegt.

7. Kosmetische Zusammensetzungen nach einem der Ansprüche 1-6, wobei das Gewichtsverhältnis zwischen Komponente a) und b) 1:1 ist.

8. Kosmetische Zusammensetzungen nach einem der Ansprüche 1 bis 7, wobei Komponente b) den Extrakt von Helianthus tuberosus und mindestens ein Verdünnungsmittel umfasst, das ausgewählt ist aus der Klasse von Polyolen.

9. Kosmetische Zusammensetzungen nach einem der Ansprüche 1 bis 8, wobei die Polyole ausgewählt sind aus Glycerin und Propandiol und jeweiligen Gemischen.

10. Kosmetische Zusammensetzungen nach einem der Ansprüche 1 bis 9, wobei die Polyole ein Gemisch aus Glycerin und Propandiol in den folgenden Gewichtsverhältnissen in Bezug auf den Extrakt Glycerin/Propandiol/Extrakt von jeweils 35/35/30 sind.

11. Verwendung von kosmetischen Zusammensetzungen nach einem der Ansprüche 1-10 zur Behandlung und Vorbeugung der extrinsischen und intrinsischen Alterung der Haut.

## Revendications

1. Compositions cosmétiques comprenant comme principe actif :
a) de l'acide hyaluronique ou un sel biologiquement acceptable de celui-ci,
b) un osmoprotecteur comprenant un extrait d'Helianthus tuberosus en combinaison avec des excipients appropriés, dans lequel
- un composant a) est dans des concentrations comprises entre 0,01 et 1 % en poids par rapport au poids total desdites compositions cosmétiques,
- un composant b) est dans des concentrations comprises entre 0,01 et 1 % en poids par rapport au poids total desdites compositions ;
- l'extrait d'Helianthus tuberosus est obtenu à partir de racines ou de tubercules de ladite plante par un processus digestif.

2. Compositions cosmétiques selon la revendication 1, dans lesquelles le sel biologiquement acceptable est un sel d'un métal alcalin.

3. Compositions cosmétiques selon les revendications 1 et 2, dans lesquelles le sel biologiquement acceptable est le sel de sodium.

4. Compositions cosmétiques selon l'une quelconque des revendications 1 à 3, dans lesquelles l'acide hyaluronique est sélectionné parmi au moins l'un des types suivants :
A) acide hyaluronique avec un poids moléculaire moyen en poids < 10 000 Da,
B) acide hyaluronique avec un poids moléculaire moyen en poids compris entre 10 000 et 1 000 000 Da,
C) acide hyaluronique avec un poids moléculaire moyen en poids > 1 000 000 Da.

5. Compositions cosmétiques selon l'une quelconque des revendications 1 à 4, qui comprennent un ou les trois types d'acide hyaluronique, A), B) et C), dans des concentrations telles que la somme des concentrations desdits types d'acide hyaluronique est comprise entre 0,01 % et 1 % en poids par rapport au poids total desdites compositions cosmétiques.

6. Compositions cosmétiques selon l'une quelconque des revendications 1 à 5, dans lesquelles le rapport en poids des composants a) et b) est compris entre 1:2 et 2:1.

7. Compositions cosmétiques selon l'une quelconque des revendications 1 à 6, dans lesquelles le rapport en poids des composants a) et b) est de 1:1.

8. Compositions cosmétiques selon l'une quelconque des revendications 1 à 7, dans lesquelles le composant b) comprend l'extrait d'Helianthus tuberosus et au moins un diluant choisi dans la classe des polyols.

9. Compositions cosmétiques selon l'une quelconque des revendications 1 à 8, dans lesquelles lesdits polyols sont choisis parmi la glycérine et le propanediol et des mélanges relatifs.

10. Compositions cosmétiques selon l'une quelconque des revendications 1 à 9, dans lesquelles lesdits polyols sont un mélange de glycérine et de propanediol dans les rapports en poids suivants par rapport à l'extrait glycérine /propanediol/extrait respectivement de 35/35/30.

11. Utilisation de compositions cosmétiques selon l'une quelconque des revendications 1 à 10 pour le traitement et la prévention du vieillissement extrinsèque et intrinsèque de la peau.
